# EUROPÄISCHE PATENTANMELDUNG

(11) **EP 1 418 172 A2**
(43) Veröffentlichungstag der Anmeldung: **12.05.2004**
(21) Anmeldenummer: 03025652.3
(22) Anmeldetag: 07.11.2003
(51) Int. Cl.: C07C 253/10

(54) **Verfahren zur CaO-katalysierten Herstellung von Isophoronnitril**

(30) Priorität: 07.11.2002 DE 10251680
(71) Anmelder: BASF AKTIENGESELLSCHAFT, 67056 Ludwigshafen (DE)
(72) Erfinder: Kunsmann-Keitel, Dagmar, Dr., 67117 Limburgerhof (DE); Braun, Gerold, Dr., 67071 Ludwigshafen (DE); Münster, Ingo, Dr., 67459 Böhl-Iggelheim (DE); Mundinger, Klaus, Dr., 67117 Limburgerhof (DE); Scherhag, Gunter, Dr., 69120 Heidelberg (DE); Siegel, Wolfgang, Dr., 67117 Limburgerhof (DE)
(74) Vertreter: Isenbruck, Günter, Dr.

(57) **Zusammenfassung**

Die vorliegende Erfindung beschreibt ein Verfahren zur Herstellung von Isophoronnitril durch Addition von HCN an Isophoron. Der dabei eingesetzte Katalysator ist CaO, das eine BET-Oberfläche von > 1,5 m²/g aufweist. Durch den Einsatz eines derartigen Katalysators werden hohe Ausbeuten und Selektivitäten erzielt.

## Beschreibung

Die vorliegende Erfindung bezieht sich auf ein Verfahren zur Herstellung von Isophoronnitril durch Anlagerung von HCN an Isophoron, bei dem als Katalysator CaO eingesetzt wird.

Die Addition von HCN an Isophoron ist eine an sich bekannte Reaktion. Sie ist in der nachfolgenden Gleichung (I) dargestellt.

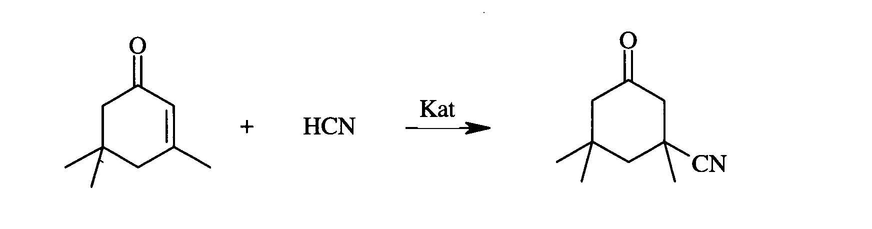

Die Reaktion verläuft in Anwesenheit eines Katalysators, dessen Eigenschaften für eine erfolgreiche Durchführung der Additionsreaktion kritisch sind. Die Aufgabe des Katalysators besteht darin, aus dem HCN Cyanidionen in einer Menge zu bilden, bei der die gewünschte Addition mit hoher Ausbeute und Selektivität verläuft. Der Katalysator muß dabei die zugeführte HCN-Menge sofort mit dem Isophoron umsetzen. Diese Menge darf nicht zu niedrig sein, da ansonsten zum Erzielen einer akzeptablen Ausbeute lange Reaktionszeiten notwendig wären. Zudem tritt bei zu langer Reaktionsdauer und niedriger Cyanidionen-Konzentration eine Zersetzung des gebildeten Isophoronnitrils zu Isophoron und HCN ein. Bei einer zu hohen Stationärkonzentration an HCN - die natürlich auch durch zu schnelles Zutropfen bzw. Vorliegen eines ungeeigneten Katalysators vorliegen kann - tritt eine Polymerisation des HCN ein, das dadurch für die weitere Reaktion verloren geht. Eine weitere unerwünschte Nebenreaktion liegt in der Dimerisierung von Isophoron zu Di-Isophoron in Gegenwart von Basen. Das Di-Isophoron kann dann, insbesondere bei hohen Reaktionstemperaturen und längerer Reaktionsdauer, polymerisieren und geht als Ausgangsstoff verloren.

Die Katalysatoren, die verwendet werden, sind generell Basen, die mit dem HCN unter Reaktionsbedingungen Cyanidionen bilden.

Aus dem Stand der Technik ist die Verwendung unterschiedlicher Basen bekannt.

Die DE-AS 1 085 871 beschreibt ein Verfahren zur Addition von HCN an cyclische Ketone, u.a. Isophoron. Die bei der Addition eingesetzten Katalysatoren sind stark alkalische, Cyanidionen bildende Katalysatoren. Dabei handelt es sich um Alkalimetalle und deren Carbonate, Erdalkalimetalle und Alkali- und Erdalkalialkoholate, -oxide, - hydroxide, -peroxide und -cyanide, tertiäre Amine und quartemäre Ammoniumbasen. Als typische Beispiele von Katalysatoren, die eingesetzt werden können, werden Natrium, Kalium, Lithium, Natriummethylat, Kaliumbutylat, Lithiumethylat, Magnesiumethylat, Natriumoxid, Kaliumhydroxid, Calciumoxid, Bariumhydroxid, Strontiumhydroxid, Natriumperoxid, Magnesiumperoxid, Kaliumcyanid, Lithiumcyanid, Bariumcyanid, Magnesiumcyanid, Natriumcarbonat, Kaliumcarbonat, Trimethylamin, Triethylamin, Triethanolamin, Octyldimethylamin, N-Methylmorpholin, Benzyltrimethylammoniumhydroxid, Dibenzyldimethylammoniumhydroxid und Dodecenyltriethylammoniumhydroxid genannt.

Die DE-AS 1 240 854 offenbart ein Verfahren zur Herstellung von Isophoronnitril durch Addition von HCN an Isophoron, bei dem ein alkalischer Katalysator in Mengen < 10⁻¹ bis 10-³ Gew.-% eingesetzt und in Abwesenheit eines Lösungsmittels gearbeitet wird. Als besonders geeignete Katalysatoren werden Alkalicyanide, -hydroxide und -alkoholate genannt, die Beispiele beschreiben die Verwendung von NaOH und Natriummethylat.

Aus der EP-A 0 443 615 ist es bekannt, die Addition von HCN an Isophoron unter Einsatz von LiOH als Katalysator durchzuführen, wodurch vorteilhafte Resultate erreicht werden sollen.

Das in der US 5,183,915 beschriebene Verfahren zur Addition von HCN an Isophoron setzt als Katalysator ein Oniumsalz von Stickstoff, Phosphor oder Arsen mit Cyanid als Gegenion ein.

Bei dem in der US 5,235,089 offenbarten Reaktion zur Herstellung von Isophoronnitril durch Addition von HCN an Isophoron sind die einsetzbaren Katalysatoren aus der Gruppe bestehend aus Lithiumhydroxid, Lithiumhydroxidmonohydrat, Lithiumcyanid oder Lösungen davon ausgewählt.

Nach der EP-A 0 554 786 wird die basenkatalysierte Umsetzung von Isophoron mit HCN in zwei getrennten Reaktionszonen durchgeführt, bei der in einer Reaktionszone eine im wesentlichen vollständige Rückvermischung und in der anderen im wesentlichen keine Rückvermischung eintritt. Als basische Katalysatoren sind alle Substanzen geeignet, die unter den Reaktionsbedingungen aus HCN Cyanidionen bilden, beispielsweise Hydroxide, Cyanide und Alkoholate der Alkali- und Erdalkalimetalle und quarternäre Ammoniumverbindungen. Vorzugsweise werden C₁-C₄-Alkalialkoholate wie Natriummethylat, Natriumethylat, Kaliummethylat, Kalium-tert.-butylat, oder Lithiummethylat eingesetzt.

Die EP-A 0 671 384 offenbart die Verwendung bestimmter organischer Ammoniumkatalysatoren bei der Umsetzung von HCN mit Isophoron zu Isophoronnitril.

Entsprechend der Lehre der EP-A 0 985 659 schließlich wird als Base bei der Addition von HCN an Isophoron das Betain 1,3-Dimethyl-imidazolium-4-carboxylat eingesetzt.

Es besteht jedoch weiterhin ein Bedarf an Katalysatoren für die Addition von HCN an Isophoron, die kostengünstig sind und hohe Ausbeuten sowie Selektivitäten liefern.

Die Aufgabe der vorliegenden Erfindung besteht in der Bereitstellung eines solchen Katalysators.

Es wurde nun gefunden, daß durch die Verwendung von CaO mit einer BET-Oberfläche von > 1,5 m²/g bei der Addition von HCN an Isophoron solch hohe Selektivitäten und Ausbeuten erzielt werden. Dadurch wird ein weiterer Katalysator für die erwähnte Reaktion zur Verfügung gestellt.

Der Katalysator, der auch in der Bauindustrie Anwendung findet, ist in großen Mengen preisgünstig zu erwerben. Weiterhin wurde gefunden, daß das eingesetzte CaO eine hohe Aktivität aufweist. So liegen im allgemeinen die erzielbaren Raum-Zeit-Ausbeuten höher als bei Katalysatoren, die preislich in etwa vergleichbar sind, wie LiOH, NaOH oder auch NaOMe. Oft liegt die Aktivität auch über der komplexer und kostspieligerer Katalysatoren, die in den vorstehend zitierten Dokumenten erwähnt sind. Schließlich liegen auch die Selektivitäten, die erzielt werden können, bei hohen Werten, etwa 98 bis 99% bezüglich HCN und 98 bis 99% bezüglich Isophoron.

Um für einen Einsatz bei der Addition von HCN an Isophoron geeignet zu sein, muß das eingesetzte CaO insbesondere eine hohe BET (Brunauer Emett Teller)-Oberfläche von > 1,5 m²/g aufweisen. Noch bessere Ergebnisse werden erhalten, wenn ein CaO einer BET-Oberfläche > 2,0 m²/g, insbesondere > 2,3 m²/g, eingesetzt wird.

Weiterhin hat es sich als vorteilhaft erwiesen, wenn das eingesetzte CaO nur einen geringen Gehalt an Verunreinigungen aufweist. Das CaO sollte eine Reinheit > 96%, vorzugsweise > 97%, insbesondere > 98% aufweisen.

Das erfindungsgemäße Verfahren wird bei einer Temperatur von 100 bis 200°C, vorzugsweise 140 bis 190°C, insbesondere 150 bis 170°C durchgeführt, wobei die Durchführung bei Normal- oder Überdruck geschehen kann. Vorzugsweise wird die Reaktion bei einem Druck von 1 bis 10 bar, insbesondere 1 bis 3 bar durchgeführt. Die eingesetzte CaO-Menge beträgt 2000 bis 10.000 ppm, vorzugsweise 3000 bis 6000 ppm, bezogen auf die Gesamtmenge der Edukte. Geeignete CaO-Qualitäten sind beispielsweise von der Firma Honeywell unter der Spezifikation E529 (BET-Oberfläche 2,7 m²/g) und der Firma Schäferkalk unter der Spezifikation PRECAL 30 S (BET-Oberfläche 2,4 m²/g) erhältlich.

Da - wie bereits erwähnt - das HCN nicht derart zugegeben werden darf, daß eine zur Polymerisation ausreichende Menge davon (die nicht zu Cyanidionen umgesetzt und nicht direkt an das Isophoron addiert) in dem Reaktionsgemisch vorliegt, darf weiterhin die Geschwindigkeit der Zudosierung von HCN zu Isophoron nicht zu hoch gewählt werden.

Aufgrund der notwendigen, niedrigen HCN-Konzentrationen ist das Vorlegen von HCN mit anschließender Zugabe von Isophoron nicht sinnvoll. Es wird also immer zumindest eine Teilmenge des Isophorons vorgelegt und das HCN bei einer Temperatur zugefügt, bei der die Addition an Isophoron mit der gewünschten Geschwindigkeit eintritt. Dabei muß auch der Katalysator anwesend sein. HCN darf auch nicht zu langsam zugegeben werden, da hierbei, insbesondere bei hohen Reaktionstemperaturen, Isophoronnitril unter Neubildung von HCN und Isophoron zerfällt.

Generell wird das erfindungsgemäße Verfahren derart durchgeführt, daß ein Überschuß an Isophoron eingesetzt wird, da so eine höhere Selektivität zu Isophoronnitril erzielt wird. Im Allgemeinen beträgt das molare Verhältnis Isophoron/HCN > 1. Für eine günstige Raum-Zeit-Ausbeute sollte der Isophoron-Überschuß so gering wie möglich gehalten werden. Dabei sollten vorzugsweise Werte von 1,2 bis 2, insbesondere 1,3 bis 1,5, eingehalten werden. Die Gesamtmenge des Isophorons kann vorgelegt und auf die gewünschte Reaktionstemperatur gebracht werden, bevor in Anwesenheit des Katalysators das HCN zugegeben wird. Es hat sich als günstig erwiesen, einen Teil des Isophorons vorzulegen und nach Erwärmen auf Reaktionstemperatur ein Gemisch aus Isophoron und HCN in einem geeigneten Verhältnis in Anwesenheit des Katalysators zuzugeben, bis die Reaktion beendet ist. Vorzugsweise liegt dieses Verhältnis bei Werten von ca. 1:2. Der Katalysator kann bereits während des Aufwärmens des Isophorons anwesend sein. Im Unterschied zu den üblichen, nach dem Stand der Technik verwendeten Katalysatoren, insbesondere Alkalisalzen, tritt dabei keine oder nur eine untergeordnete Polymerisation des Isophorons ein. Es ist jedoch bevorzugt, einen Teil des Isophorons auf Reaktionstemperatur aufzuheizen und danach das CaO zuzugeben. So läßt sich gegebenenfalls die Polymerisation des Isophorons weiter unterdrücken. Optionsweise wird bei dieser Durchführungsweise eine geringe Menge des Isophoron/HCN-Gemischs bereits gemeinsam mit dem Katalysator zugegeben.

Zu dem die gewünschte Reaktionstemperatur aufweisenden Isophoron/CaO-Gemisch wird dann, je nach gewählter Ausführungsform, HCN oder ein Isophoron/HCN-Gemisch gegeben.

Innerhalb der vorstehend dargelegten Reaktionsparameter wird das HCN bzw. das Isophoron/HCN-Gemisch so zudosiert, daß eine ausreichend niedrige Stationärkonzentration von HCN resultiert und eine hohe Selektivität sowie ein hoher Umsatz zu Isophoronnitril erzielt werden. Dabei darf nur eine geringe Polymerisation des HCN auftreten, da hierdurch der Umsatz und die Selektivität sinken würden. Die genauen Bedingungen, die eingehalten werden müssen, können vom Fachmann durch Routineversuche ermittelt werden. Vorzugsweise werden die stationären Konzentrationen an nicht umgesetztem, freiem HCN und die Gesamtkonzentration an Cyanidionen (Summe aus freiem HCN und als Cyanhydrine von Isophoron und Isophoronnitril gebundenem Cyanid) bestimmt und die Reaktionsbedingungen angepaßt, bis die Werte in dem gewünschten Rahmen liegen. Die Bestimmung der genannten Konzentrationen erfolgt vorzugsweise durch die Bestimmungen nach Volhard bzw. Liebig.

Das Verfahren kann diskontinuierlich, kontinuierlich oder semi-kontinuierlich durchgeführt werden, wobei die semi-kontinuierliche Durchführungsform bevorzugt ist.

Das Verfahren kann mit oder ohne Zusatz eines geeigneten Lösungsmittels durchgeführt werden. Vorzugsweise wird die Reaktion ohne Lösungsmittel durchgeführt. Für den Fall, daß ein Lösungsmittel eingesetzt wird, eignen sich Toluol und/oder Dimethylformamid (DMF), vorzugsweise DMF.

Die Aufarbeitung des Reaktionsgemisches nach beendeter Reaktion geschieht auf übliche, dem Fachmann bekannte Weise. Vorzugsweise wird überschüssiges Isophoron durch Destillation abgetrennt und vorteilhafterweise erneut eingesetzt. Danach wird das gebildete Isophoronnitril, vorzugsweise ebenfalls destillativ, von Hochsiedern und eingesetztem Katalysator getrennt.

Die Erfindung wird nun in den nachfolgenden Beispielen veranschaulicht, in denen RZA Raum-Zeit-Ausbeute, IP Isophoron und IPN Isophoronnitril bedeuten.

### Beispiele

### Beispiel 1

Isophoron (622 g, 4,5 mol) wird in einem Rührkessel vorgelegt und auf 150°C erhitzt. Bei Erreichen der Reaktionstemperatur wird CaO (Honeywell E529; 4,14 g; 4500 ppm bezogen auf den Gesamtansatz) zugegeben und mit der Dosierung eines Isophoron/HCN-Gemischs (81 g HCN, 3 mol; 207,3 g IP 1,5 mol) begonnen. Nach 60 Minuten ist das Gemisch zudosiert und das Reaktionsgemisch wird noch für eine weitere Stunde auf Reaktionstemperatur gehalten. Der HCN-Umsatz beträgt 99,5%.
Zur Bestimmung der Ausbeute und der Selektivität wird der Reaktionsansatz bei 0,1 mbar fraktioniert destilliert. Ausbeute an IPN bezogen auf HCN: 98,7%; Selektivität bezogen auf IP: 97,1%.

### Beispiel 2 (Vergleichsbeispiel)

Isophoron (865,2 g, 6,27 mol) wird in einem Rührkessel vorgelegt und auf 150°C erhitzt. Bei Erreichen der Reaktionstemperatur wird 20%ige NaOH (5,75 g, 0,34 mol% bezogen auf IP) zugegeben und mit der Dosierung von Isophoron/HCN-Gemisch (113,0 g HCN, 4,18 mol; 288,5 g IP 2,09 mol) begonnen. Nach 300 Minuten ist das Gemisch zudosiert und die Reaktion wird noch für eine weitere Stunde auf Reaktionstemperatur gehalten. Der HCN-Umsatz beträgt 100%.
Zur Bestimmung der Ausbeute und der Selektivität wird der Reaktionsansatz bei 0,1 mbar fraktioniert destilliert. Ausbeute an IPN bezogen auf HCN: 94,1%; Selektivität bezogen auf IP: 96,3%.

### Beispiel 3 (Vergleichsbeispiel)

Isophoron (865,2 g 6,27 mol) wird in einem Rührkessel vorgelegt und auf 150°C erhitzt. Bei Erreichen der Reaktionstemperatur wird LiOH (0,98 g, 0,48 mol% bezogen auf IP) zugegeben und mit der Dosierung vom Isophoron/HCN-Gemisch (113,0 g HCN, 4,18 mol; 288,5 g IP 2,09 mol) begonnen. Nach 300 Minuten ist das Gemisch zudosiert und die Reaktion wird noch für 2,7 weitere Stunden auf Reaktionstemperatur gehalten. Der HCN-Umsatz beträgt 99,5%.

Zur Bestimmung der Ausbeute und der Selektivität wird der Reaktionsansatz bei 0,1 mbar fraktioniert destilliert. Ausbeute an IPN bezogen auf HCN: 80,5%; Selektivität bezogen auf IP: 95,4%.

Wie die Beispiele 1 bis 3 zeigen, sind die erzielten Selektivitäten und Ausbeuten mit CaO am besten. Die längeren Dosierzeiten bei NaOH und LiOH weisen zusätzlich auf eine geringere Aktivität des Katalysators hin.

### Beispiel 4

Es wurden Reihenversuche mit CaO (Honeywell E529) als Katalysator zum Auffinden der besten Reaktionsbedingungen durchgeführt. Dabei wurde ein Teil des im Überschuß eingesetzten Isophorons vorgelegt, ein Teil im Gemisch mit HCN zudosiert. Dabei wurde bei allen Versuchen die gleiche Menge an Zulauf (103,5 g IP, 0,75 mol; 40,5 g HCN, 1,5 mol) verwendet. Die restliche Menge an Isophoron (variabel) diente als Vorlage.

Zu dem vorgelegten IP wurde bei Erreichen der Reaktionstemperatur CaO zugegeben und mit der Dosierung des IP/HCN-Gemisches über verschiedene Zeiträume begonnen. Die Gesamtreaktionszeit betrug 180 Minuten.

Die Selektivitäten bezogen auf beide Einsatzstoffe wurden anschließend gaschromatographisch bestimmt. Die Ergebnisse sind in der nachfolgenden Tabelle 1 wiedergegeben.

**Tabelle 1:**

| Umsetzung von Isophoron mit HCN bei verschiedenen Reaktionsbedingungen | | | | | | |
|---|---|---|---|---|---|---|
| Nr. | T[°C] | Molverhältnis Isophoron/HCN | Dosierzeit [min] | CaO [ppm] | S (HCN) | S (IP) |
| 1 | 155 | 1,5 | 100 | 3750 | 99,0 | 97,7 |
| 2 | 155 | 1,33 | 120 | 3750 | 98,1 | 97,5 |
| 3 | 165 | 1,2 | 84 | 4100 | 95,9 | 99,9 |
| 4 | 165 | 1,33 | 84 | 3750 | 97,5 | 99,0 |
| 5 | 175 | 1,33 | 80 | 3750 | 97,1 | 98,2 |
| 6 | 175 | 1,33 | 65 | 3750 | 97,3 | 98,6 |

## Patentansprüche

1. Verfahren zur Herstellung von Isophoronnitril durch Addition von HCN an Isophoron, **dadurch gekennzeichnet, daß** CaO mit einer BET-Oberfläche von > 1,5 m²/g als Katalysator verwendet wird.

2. Verfahren nach Anspruch 1, **dadurch gekennzeichnet, daß** das eingesetzte CaO eine BET-Oberfläche von > 2 m²/g, vorzugsweise > 2,3 m²/g aufweist.

3. Verfahren nach Anspruch 1 oder 2, **dadurch gekennzeichnet, daß** das CaO eine Reinheit von > 96%, vorzugsweise > 97%, insbesondere > 98%, aufweist.

4. Verfahren nach einem der Ansprüche 1 bis 3, **dadurch gekennzeichnet, daß** die Reaktion bei Temperaturen von 100 bis 200°C, vorzugsweise 140 bis 190°C, insbesondere 150 bis 170°C, durchgeführt wird.

5. Verfahren nach einem der Ansprüche 1 bis 4, **dadurch gekennzeichnet, daß** die Reaktion bei einem Druck von ≥ 1 bar, vorzugsweise 1 bis 10 bar, insbesondere 1 bis 3 bar, durchgeführt wird.

6. Verfahren nach einem der Ansprüche 1 bis 5, **dadurch gekennzeichnet, daß** das Verhältnis Isophoron/HCN bei Werten von ≥ 1, vorzugsweise 1 bis 2, insbesondere 1, 3 bis 1,5, liegt.

7. Verfahren nach einem der Ansprüche 1 bis 6, **dadurch gekennzeichnet, daß** Isophoron und CaO gemeinsam vorgelegt und vor der Zugabe des HCN auf Reaktionstemperatur gebracht werden.

8. Verfahren nach einem der Ansprüche 1 bis 6, **dadurch gekennzeichnet, daß** Isophoron alleine vorgelegt und auf Reaktionstemperatur erhitzt wird, bevor CaO zugegeben wird, anschließend das HCN zugegeben wird.

9. Verfahren nach einem der Ansprüche 1 bis 8, **dadurch gekennzeichnet, daß** ein Teil des Isophorons vorgelegt und ein Gemisch aus Isophoron und HCN zugegeben wird.

10. Verfahren nach einem der Ansprüche 1 bis 9, **dadurch gekennzeichnet, daß** die Konzentration des Katalysators 2000 bis 10.000 ppm, vorzugsweise 3000 bis 6000 ppm, bezogen auf die Gesamtmenge der Edukte, beträgt.
